# EUROPEAN PATENT APPLICATION

(11) **EP 4 487 755 A1**
(43) Date of publication of application: **08.01.2025**
(21) Application number: 23902612.3
(22) Date of filing: 08.12.2023
(51) Int. Cl.: A61B 3/13

(54) **SURGICAL MICROSCOPE SYSTEM AND SURGICAL MICROSCOPE**

(30) Priority: 12.12.2022 CN 202211587632
(71) Applicant: Towardpi (Beijing) Medical Technology Ltd., Beijing 102206 (CN)
(72) Inventor: LU, Lirong, Beijing 102206 (CN); WANG, Xiao, Beijing 102206 (CN)
(74) Representative: Murgitroyd & Company
(86) International application number: PCT/CN2023/137512
(87) International publication number: WO 2024/125416

(57) **Abstract**

Disclosed herein are a surgical microscope system and a surgical microscope. The surgical microscope system includes a microscope imaging module (01) and a lighting module (02), wherein: the microscope imaging module (01) includes an objective lens (11), a dichroic beam splitting lens (12), a zoom unit (13), a beam splitter (14), a lens tube (15) and an eyepiece set (16) disposed along a main optical axis (L1); the lighting module (02) includes a coaxial lighting unit (21) and an angled lighting unit (22), the coaxial lighting unit (21) includes a first light source (211) and a first field stop (212), the first field stop (212) is disposed between a first light source (211) and a dichroic beam splitting lens (12), and coaxial lighting beams emitted from the first light source (211) pass through the first field stop (212), are reflected by the dichroic beam splitting lens (12), then pass through the objective lens (11), and reach an observed object surface (M) along a direction of a main optical axis (L1) and form a first optical spot; angled lighting beams emitted from the angled lighting unit (22) are reflected by the dichroic beam splitting lens (12), then pass through the objective lens (11), and reach the observed object surface (M) along a direction including a preset angle with respect to the main optical axis (L1) and form a second optical spot; the first optical spot is adjustable in size.

## Description

The present disclosure claims the priority from the CN patent application No. 202211587632.3 filed with the China National Intellectual Property Administration (CNIPA) on December 12, 2022, the contents of which are hereby incorporated by reference in their entirety.

### TECHNICAL FIELD

The present disclosure relates to the field of optical technologies and surgical microscopes, and specifically to a surgical microscope system and a surgical microscope applicable to ophthalmic surgery.

### BACKGROUND

Since legacy surgical microscopes have simple functions and provide surgeons with less comprehensive and limited reference data and images, it poses great challenges for ophthalmologists to observe tiny details of patients' eyes during ophthalmic surgery using the legacy surgical microscopes.

For example, vitreoretinal surgery performed on eyes involves a vitrectomy (i.e., removing a vitreous body from the posterior chamber to access the retina). For a successful vitrectomy, it is generally required to remove the vitreous body from the posterior chamber, including removing a challenging area near the vitreous base. In the circumstance, the transparency of the vitreous body poses a great challenge to perform vitrectomy depending only on the legacy surgical microscope.

### SUMMARY

The present disclosure provides a surgical microscope system and a surgical microscope, for example, which can be applied to ophthalmic operations.

In a first aspect of the present disclosure, there is provided a surgical microscope system, comprising: a microscope imaging module and a lighting module;
the microscope imaging module comprises an objective lens, a dichroic beam splitting lens, a zoom unit, a beam splitter, a lens tube and an eyepiece set disposed along a main optical axis; beams from an observed object surface pass sequentially through the objective lens, the dichroic beam splitting lens and the zoom unit and are then split by the beam splitter into first beams and second beams, wherein the first beams pass sequentially through the lens tube and the eyepiece set along the main optical axis, and the observed object surface is configured to be observed by an observer; the microscope imaging module further comprises an image acquisition unit disposed on a traveling path of the second beams and configured to acquire surgical images; wherein the first beams and the second beams are different in traveling direction;
the lighting module comprises a coaxial lighting unit and an angled lighting unit which are located on a side of the dichroic beam splitting lens facing the objective lens; the coaxial lighting unit comprises a first light source and a first field stop disposed between the first light source and the dichroic beam splitting lens, and coaxial lighting beams emitted from the first light source pass through the first field stop, are reflected by the dichroic beam splitting lens, then pass through the objective lens, and reach the observed object surface along a direction of the main optical axis and form a first optical spot; angled lighting beams emitted from the angled lighting unit are reflected by the dichroic beam splitting lens, then pass through the objective lens, and reach the observed object surface along a direction including a preset angle with respect to the main optical axis and form a second optical spot; the first optical spot is adjustable in size.

In a second aspect of the present disclosure, there is also provided a surgical microscope, comprising a surgical microscope system according to any of the embodiments in the first aspect of the present disclosure.

### BRIEF DESCRIPTION OF THE DRAWINGS

Fig. 1 is a schematic diagram of a surgical microscope system applicable to ophthalmic surgery provided by embodiments of the present disclosure;
Fig. 2 is a schematic diagram of a first field stop provided by embodiments of the present disclosure;
Fig. 3 is a schematic diagram of another first field stop provided by embodiments of the present disclosure;
Fig. 4 is a schematic diagram of a further first field stop provided by embodiments of the present disclosure;
Fig. 5 is a schematic diagram of a fundus functional lens provided by embodiments of the present disclosure;
Fig. 6 is a schematic diagram of a zoom unit provided by embodiments of the present disclosure; and
Fig. 7 is a schematic diagram of an optical path of a scanning unit provided by embodiments of the present disclosure.

The reference signs employed therein are listed below:
L1 - main optical axis; M - observed object surface; 01 - microscope imaging module; 02 - lighting module; 03 - Optical Coherence Tomography (OCT) imaging module; 11 - objective lens; 111 - first lens; 112 - second lens; 12 - dichroic beam splitting lens; 13 - zoom unit; 14 - beam splitter; 15 - lens tube; 16 - eyepiece set; 17 - image acquisition unit; 18 - first reflector; S3 - first beam; S4 - second beam; 21 - coaxial lighting unit; 211 - first light source; 212 - first field stop; 2121 - first disc-shaped stop; 2122 - rectangular stop; 2123 - second disc-shaped stop; O - clear aperture; 213 - fundus functional lens; P1 - light transmitting portion; P2 - light shielding portion; 214 - first lighting lens set; 22 - angled lighting unit; 221 - second light source; 222 - second field stop; 223 - second lighting lens set; 23 - stray light absorption unit; 120 - front fixed set; 121 - third lens; 122 - fourth lens; 130 - zoom set; 131 - fifth lens; 132 - sixth lens; 140 - compensation set; 141 - seventh lens; 142 - eighth lens; 150 - rear fixed set; 151 - first lens set ; 152 - second lens set; 1501 - cemented lens; 1502 - meniscus lens; 31 - scanning unit; 311 - scanning galvanometer; 312 - focus tuning lens; 313 - OCT system light source; 314 - second reflector; 315 - focusing lens; 32 - OCT image acquisition unit.

### DETAILED DESCRIPTION OF EMBODIMENTS

Reference will now be made to the drawings according to the embodiments of the present disclosure to describe the technical solution of the present disclosure. The embodiments depicted therein are only a part of the embodiments of the present disclosure.

In some embodiments, in order to solve the problem that the legacy surgical microscopes have limited functions, OCT imaging is provided for ophthalmic surgery. However, in those embodiments, OCT images can be applied only before surgery and cannot be combined with a surgical microscope during surgery, thus providing ophthalmologists with quite limited assistance.

In some embodiments, for a surgical microscope for ophthalmic surgery, a lighting module includes a coaxial lighting unit and an angled lighting unit. Wherein, the angled lighting unit is also referred to as field lighting unit, and a lighting path of a midfield lighting unit used in the surgical microscope can provide illumination from different angles, so as to provide the desired ambient light for the whole surgical field. The coaxial lighting unit, also referred to as 0° lighting unit, can provide crucial background illumination for the surgical field that is limited by the pupil area of the crystalline lens during cataract surgery, which meets the basic requirement of an optimal lighting system during cataract surgery. That is, the coaxial lighting unit can meet the requirement on the background light in the surgical area that requires uniform red reflex with a high contrast. However, in some embodiments according to the related technologies, the fundus optical spot (also referred to as fundus light dot) generated by the coaxial lighting unit cannot be adjusted in size.

However, during cataract surgery, if there is only a small diseased area on a patient's fundus, or if a patient has a small fundus (e.g., a child patient typically has a small fundus), a smaller fundus spot can provide a higher contrast. In addition, in a case of thicker cataracts, "red reflex" may be too dim for the cataract surgery. In the circumstance, if the fundus is brightened by enlarging the fundus spot, it is helpful for the cataract surgery.

Furthermore, in some embodiments according to the related technologies, the coaxial lighting unit and the angled lighting unit in the lighting module of the surgical microscope for ophthalmic surgery can be enabled only separately, not simultaneously.

However, during ophthalmic surgery, in some circumstances, the field of view needs to be broadened to facilitate the surgery; in some other circumstances where an assistant has to help the surgeon to treat the area around the eye, it is required to enable simultaneously the coaxial lighting unit and the angled lighting unit.

Therefore, in order to cause the surgical microscope to better meet the requirements of surgical operations, in particular ophthalmic operations, an OCT imaging module is added to the surgical microscope in some embodiments of the present disclosure, thus enabling an OCT imaging function during the ophthalmic operations, which breaks the limit of using OCT imaging only before the ophthalmic operations. In some further embodiments of the present disclosure, the lighting module in the surgical microscope is improved such that the size of the optical spot formed by the coaxial lighting unit at the fundus can be adjusted. In some other embodiments of the present disclosure, the lighting module of the surgical microscope is improved such that the coaxial lighting unit and the angled lighting unit can be enabled not only separately but also simultaneously.

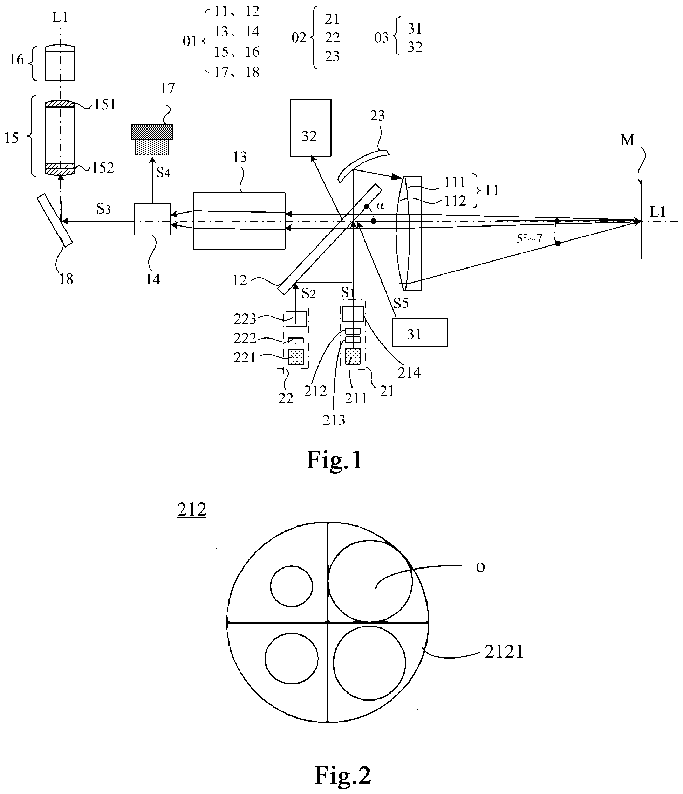

Fig. 1 is a schematic diagram of a surgical microscope system for ophthalmic surgery. As shown therein, the embodiments of the present disclosure provide a surgical microscope system which can be applied to medical ophthalmic surgery. The surgical microscope system includes a microscope imaging module 01 and a lighting module 02; the microscope imaging module 01 includes an objective lens 11, a dichroic beam splitting lens 12, a zoom unit 13, a beam splitter 14, a lens tube 15 and an eyepiece set 16 arranged along a direction of a main optical axis L1 from an object surface to an image surface; beams from an observed object surface M pass sequentially through the objective lens 11, the dichroic beam splitting lens 12 and the zoom unit 13, and are then split by the beam splitter 14 into first beams S3 and second beams S4, where the first beams S3 pass sequentially through the lens tube 15 and the eyepiece set 16 along the main optical axis L1, which is configured to be observed by an observer; the microscope imaging module 01 further includes an image acquisition unit 17 which is located on a traveling path of the second beams S4 and configured to acquire surgical images; wherein the first beams S3 and the second beams S4 are different in traveling direction; the lighting module 02 includes a coaxial lighting unit 21 and an angled lighting unit 22, where the coaxial lighting unit 21 and the angled lighting unit 22 are located on the same side of the dichroic beam splitting lens 12 facing the objective lens 11; the coaxial lighting unit 21 includes a first light source 211, a first field stop 212 and a first lighting lens set 214, the first field stop 212 is disposed between the first light source 211 and the dichroic beam splitting lens 12 and between the first light source 211 and the first lighting lens set 214, and in a case where the coaxial lighting unit 21 further includes a fundus functional lens 213, the first field stop 212 is disposed between the fundus functional lens 213 and the first lighting lens set 214. The coaxial lighting beams S1 emitted from the first light source 211 pass through the first field stop 212 and the first lighting lens set 214, then pass through the objective lens 11 after reflected by the dichroic beam splitting lens 12, and finally reach the observed object surface M along the direction of the optical main axis L1 and form a first optical spot (i.e., a fundus optical spot), where the size of the first optical spot can be adjusted; the angled lighting beams S2 emitted from the angled lighting unit 22 pass through the objective lens 11 after reflected by the dichroic beam splitting lens 12, and finally reach the observed object surface M along the direction including a preset angle (e.g. 5° to 7°) with respect to the main optical axis L1 and form a second optical spot.

In the embodiments of the present disclosure, the microscope imaging module 01 is configured to image an observed eye in a magnified manner, the lighting module 02 is configured to illuminate the optical path of the surgical microscope system, and the observed object surface M is an object surface where the retina of the observed eye is located. With respect to the main optical axis L1 of the microscope imaging module 01, the microscope imaging module 01 includes an objective lens 11, a dichroic beam splitting lens 12, a zoom unit 13, a beam splitter 14, a first reflector 18, a lens tube 15 and an eyepiece set 16 arranged along the main optical axis L1 from the object surface to the image surface, where the zoom unit 13 is configured to adjust the size of the displayed image of the observed object surface M observed through the eyepiece set 16. The microscope system is provided therein with a lighting module 02 for providing coaxial lighting and/or angled lighting for the observed eye, where the coaxial lighting refers to that the lighting beams are parallel to the main optical axial L1, and the angled lighting refers to that the lighting beams contain an angle, for example, 5° to 7°, with respect to the main optical axial L1.

By way of example, as shown in Fig. 1, both the coaxial lighting unit 21 and the angled lighting unit 22 are disposed on the same side of the dichroic beam splitting lens 12 facing the objective lens 11. The first light source 211 may be a white light source and can be configured to emit coaxial lighting beams S1. The first field stop 212 is disposed between the first light source 211 and the dichroic beam splitting lens 12. The central optical axis of the coaxial lighting beams S1 emitted from the first light source 211 illuminates an overlapping area between the dichroic beam splitting lens 12 and the main optical axis L1, such that the coaxial lighting beams S1 can pass through the objective lens 11 along the main optical axis L1 after reflected by the dichroic beam splitting lens 12, and then reach the observed object surface M and form the first optical spot. The size of the first optical spot can be adjusted by adjusting the size of the clear aperture of the first field stop 212 according to the size of the patient's fundus and/or the size of the lesion on the fundus, to meet different illumination needs.

In the embodiments of the present disclosure, since the size of the optical spot formed by the coaxial lighting unit on the fundus can be adjusted, the fundus optical spot can be reduced during cataract surgery if there is only a small diseased area on a patient's fundus, or if a patient has a small fundus, so as to provide a higher contrast and facilitate the surgery. In a case of thicker cataracts which result in too dim "red reflex," the fundus optical spot can be enlarged to brighten the fundus, which is helpful for the cataract surgery.

In the embodiments of the present disclosure, the angled lighting beams S2 emitted from the angled lighting unit 22 illuminate an area deviating from the overlapping area between the dichroic beam splitting lens 12 and the main optical axis L1, such that the angled lighting beams S2 pass through the objective lens 11 after reflected by the dichroic beam splitting lens 12, and then reach the observed object surface M and form a second optical spot. In the embodiments of the present disclosure, by adjusting the posture of the angled lighting unit 22 can be obtained an angle from 5° to 7° between the traveling direction of the angled lighting beams S2 passing through the objective lens 11 after reflected by the dichroic beam splitting lens 12 and the traveling direction of the coaxial lighting beams S1 passing through the objective lens 11 after reflected by dichroic beam splitting lens 12, thus obtaining a larger field lighting optical spot diameter, illuminating the observed eye in a broader range, and meeting the illumination needs of ophthalmic surgery.

In an embodiment of the present disclosure, the eyepiece set 16 may be a lens set with a 10X magnification comprised of a single lens and a double cemented lens. The surgical microscope includes two imaging eyepieces respectively for a left eye and a right eye, which are arranged symmetrically. The microscope imaging module 01 further includes a first reflector 18, where the first reflector 18 is disposed between the beam splitter 14 and the lens tube 15, as shown in Fig. 1, and configured to adjust the traveling direction of the light to meet the observation need of an observer (e.g. an ophthalmologist).

A part of beams reflected by the observed object surface M enter the microscope system via the objective lens 11 along the direction of the main optical axis L1, and are split by the beam splitter 14 into first beams S3 and second beams S4 after sequentially passing through the dichroic beam splitting lens 12 and the zoom unit 13, where: the first beams S3 pass sequentially through the first reflector 18, the lens tube 15 and the eyepiece set 16, to allow the surgeon to observe the patient's eye; the second beams S4 reach the image acquisition unit 17 along another traveling direction, to generate a surgical image within the field of view of the surgical microscope for intraoperative observation and postoperative archiving. The acute angle between the dichroic beam splitting lens 12 and the main optical axis L1 is set as α. When the coaxial lighting unit 21 and the angled lighting unit 22 are fixed, the contact area between the angled lighting beams S2 and the dichroic beam splitting lens 12 is changed by adjusting the size of α, such that the angle between the angled lighting beams S2 after refracted by the objective lens 11 and the main optical axis L1. By way of example, the diameter of the second optical spot can be enlarged by increasing the angle α, so as to broaden the angled lighting range; the dimeter of the second optical spot can be reduced by decreasing the angle α, so as to narrow the angled lighting range. By adjusting the angled lighting range, the surgical requirement for the field of view can be satisfied.

According to the embodiments of the present disclosure, in the surgical microscope system, the fundus optical spot (i.e., the first optical spot described above) formed by the coaxial lighting unit of the lighting module can be set as an optical spot adjustable in size, to meet the needs of different ophthalmic operations while reducing the difficulty of the ophthalmic operations. By changing the inclined angle of the dichroic beam splitting lens with respect to the main optical axis in the surgical microscope system, the size of the lighting optical spot (i.e., the second optical spot described above) formed by the angled lighting unit in the lighting module can be adjusted, to provide different lighting ranges to meet different ophthalmic requirements and offer convenience for the surgery.

Figs. 2-4 are schematic diagrams of three types of first field stop according to embodiments of the present disclosure.

In the embodiments of the present disclosure, the first field stop 212 is provided thereon with a plurality of clear apertures O differing in size, by means of which first optical spots having different sizes can be formed.

In the embodiments of the present disclosure, since the first field stop 212 is provided thereon with a plurality of clear apertures O differing in diameter, the clear apertures O with different diameters can be selected for different surgical scenarios to adjust the size of the fundus optical spot of the examined object, thus affecting the intensity (brightness) and uniformity of the "red reflex." The red reflex testing is used to detect an abnormality of a posterior segment of an eye and an opacity along the visual axis, for example, a cataract and a corneal opacity. A greater diameter of the fundus optical spot formed by lighting indicates more uniform and brighter "red reflex," a smaller diameter of the fundus optical spot formed by lighting indicates a better contrast of the "red reflex." If only coaxial lighting is required by the surgical microscope system, the first light source 211 is turned on, and a clear aperture O with a suitable diameter is selected according to the size of the patient's fundus. The coaxial lighting beams S1 emitted from the first light source 211 pass through the clear aperture O, then through the objective lens 11 after reflected by the dichroic beam splitting lens 12, and finally reach the observed eye and form a first optical spot having a corresponding size on the fundus. In this way, the size of the lighting optical spot on the fundus can be controlled.

In the embodiments of the present disclosure, the first field stop 212 can be represented in various forms. In a possible implementation, as shown in Fig. 2, the first field stop 212 may be: a first disc-shaped stop 2121. The first disc-shaped stop 2121 is provided thereon with a plurality of clear apertures O differing in size, where centers of the plurality of clear apertures O are all located on the same circumference with the center of the first disc-shaped stop 2121 being the center. In this way, after any of the clear aperture O is moved into the coaxial lighting path by rotating the first field stop 212, the main optical axis of the coaxial lighting beams S1 emitted from the first light source 211 can be always perpendicular to the plane where the clear aperture O is located, and pass accurately through the center of the clear aperture O, to fulfil the purpose of accurate positioning and convenient adjustment.

By way of example, as shown in Fig. 2, a clear aperture O having a different diameter is disposed in each of the four quadrants of the circular first field stop 212, where the centers of the four clear apertures O are distributed in a direction with an angle less than 45° in the respective quadrants while simultaneously located on the same circumference with the center of the first field stop 212 being the center. During use, the first field stop 212 can rotate respectively angles of 45°, 135°, 225° and 270° about an axis perpendicular to the surface of the first field stop 212, which passes through the center of the first field stop 212, to move different clear apertures O into the coaxial lighting path. After any of the clear apertures O on the first field stop 212 is moved into the coaxial lighting path, the main optical axis of the coaxial lighting beams S emitted from the first light source 211 can be caused to be perpendicular to the plane where the clear aperture O is located, and accurately pass through the center of the clear aperture O. The user can select the size of the clear aperture O of the first field stop 212 according to the size of the patient's fundus, to improve the contrast of the microscope imaging.

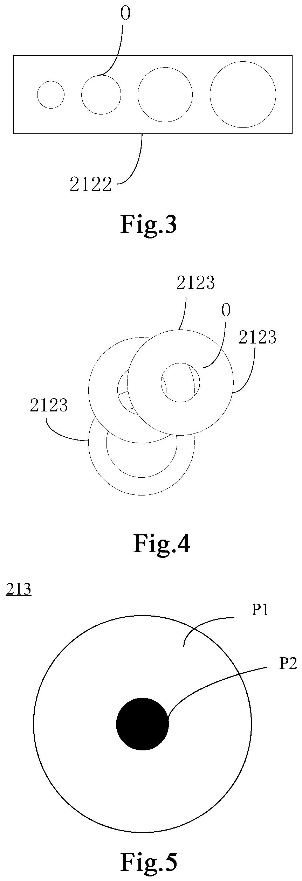

Alternatively, in another possible implementation, as shown in Fig. 3, the first field stop 212 may be a rectangular stop 2122. The rectangular stop 2122 is provided with a plurality of clear apertures O differing in size, where centers of the plurality of clear apertures O are located on the same straight line. In this way, after any of the clear aperture O is moved into the coaxial lighting path by pushing the first field stop 212, the main optical axis of the coaxial lighting beams S1 emitted from the first light source 211 can be always perpendicular to the plane where the clear aperture O is located, and pass accurately through the center of the clear aperture O, to accomplish the purpose of accurate positioning and convenient adjustment.

By way of example, the rectangular first field stop 212 as shown in Fig. 3 is provided sequentially with four clear apertures O differing in size along the long axis direction. During use, the first field stop 212 can be pushed along the long side direction of the first field stop 212, to push a clear aperture O meeting the surgical need into the coaxial lighting path. In the embodiments, after any of the clear apertures O of the rectangular first field stop 212 is pushed into the coaxial lighting path, the main optical axis of the coaxial light beams S1 emitted from the first light source 211 can always be perpendicular to the plane where the clear aperture O is located, and pass accurately through the center of the clear aperture O. The user can select the size of the clear aperture O of the first field stop 212 according to the size of the patient's fundus, thus improving the contrast of microscopic imaging.

Alternatively, in a further possible implementation, as shown in Fig. 4, the first filed stop 212 may include a plurality of second disc-shaped stops 2123 arranged in an overlaid state, where each second disc-shaped stop 2123 is provided thereon with a clear aperture O, different second disc-shaped stops 2123 each are provided with a clear aperture O with a different size, and the centers of the plurality of clear apertures O corresponding to the plurality of second disc-shaped stops 2123 in the overlaid state are located on the same axis.

By way of example, as shown in Fig. 4, each second disc-shaped stop 2123 is provided with a clear aperture O, a plurality of clear apertures O are different in size, a plurality of second disc-shaped stops 2123 is arranged in an overlaid state, and centers of the plurality of clear apertures O corresponding to the plurality of second disc-shaped stops 2123 in the overlaid state are located on the same axis. During use, a second disc-shaped stop 2123 meeting the surgical need is selected from the first field stop 212 and then moved into the coaxial lighting path, and meanwhile, the second disc-shaped stops 2123 not selected are overlaid, to save the space. After the first field stop 212 is operated to move any of the clear apertures O into the coaxial lighting path, the main optical axis of the coaxial lighting beams S1 emitted from the first light source 211 can be always perpendicular to the plane where the clear aperture O is located, and pass accurately through the center of the clear aperture O, t to accomplish the purpose of accurate positioning and convenient adjustment. The user can select the size of the clear aperture of the first field stop 212 according to the size of the patient's fundus, thus improving the contrast of microscopic imaging.

Returning to Fig. 1, the lens tube 15 is provided therein with a first lens set 151 located on an end of the lens tube 15 close to the eyepiece set 16, and a second lens set 152 located on an end of the lens tube 15 close to the zoom unit 13, where the first lens set 151 includes a meniscus lens, and the second lens set 152 includes a cemented lens.

By way of example, the lens tube 15 at two ends is provided with the first lens set 151 and the second lens set 152, respectively, where the first lens set 151 on the end close to the eyepiece set 16 is a meniscus lens. The meniscus lens may be a negative meniscus lens, and the convex surface thereof faces the eyepiece set 16 to converge beams from the zoom unit 13, thereby reducing the spherical aberration of the microscope imaging optical path. The design of the meniscus lens is helpful for compressing the numerical apertures (NA) of the zoom unit 13 and the lens tube 15, thereby minimizing the overall size of the device and further reducing the surgical space occupied by the surgical microscope. The second lens set 152 on the end close to the zoom unit 13 is a cemented lens, which can eliminate the return loss on both surface of the lens and prevent total internal reflection on the air gap, thus correcting off-axis image quality and axial chromatic aberration. By way of example, the focal length of the second lens set 152 may be set to 170mm, which is helpful for the compressed beams to enter into the eyepiece set 16.

NA is a product of the refractive index (n) and a sine value of half an aperture angle (2β) of a medium between the lens and the observed object, which is expressed with the following equation: NA = n * sinβ. The angular aperture, also referred to as "aperture angle," is an angle formed between the object point on the optical axis of the lens and the effective diameter of the front lens of the objective lens. A larger angular aperture indicates a greater luminous flux entering the lens. The angular aperture is proportional to the effective diameter of the lens while inversely proportional to a distance to the focal point.

Fig. 5 is a schematic diagram of a fundus functional lens provided by embodiments of the present disclosure.

As shown in Fig. 5, the coaxial lighting unit 21 further includes a fundus functional lens 213; the fundus functional lens 213 includes a light transmitting portion P1 and a light shielding portion, where the light transmitting portion P1 is disposed around the light shielding portion P2, the fundus functional lens 213 is disposed between the first light source 211 and the first filed stop 212, and the light shielding portion P2 is located on the optical axis of the coaxial lighting beams S1 emitted from the first light source 212. The light transmittance of the light shielding portion P2 is T1, and the light transmittance of the light transmitting portion P1 is T2, where T1<1%, and T2> 99%.

By way of example, the first light source 211 may be a white light source, and a fundus functional lens 213 is disposed between the first light source 211 and the first field stop 212, to avoid damaging the observed eye due to a too powerful light source. In these embodiments, the fundus functional lens 213 may be a flat lens of a circular shape, the light shielding portion P2 is disposed at the center of the fundus functional lens 213, and the size of the shielding portion P2 can be adjusted according to the factors such as the intensity of the light source, the coaxial illumination field of view, the observer's feeling to the light stimulation. For example, the center of the fundus functional lens 213 may be coated with a black light-absorbing material with a dimeter of 1mm, and the black area has a diameter of about 15mm after magnified by the lighting lens. The black area has a light transmittance of T1 less than 1%; the light transmitting portion P1 is disposed around the light shielding portion P2; the light transmitting portion P1 has a light transmittance of T2 greater than 99%. With the arrangement of the fundus functional lens 213 described above, the embodiments of the present disclosure can protect the pupil of the observed person from being damaged by intense light. The technical solution according to the embodiments is particularly suitable for children and adolescent patients, which can prevent a pupil from being damaged by intense light.

Returning to Fig. 1, the angled lighting unit 22 includes a second light source 221, a second field stop 222 and a second lighting lens set 223, where the second light source 221 is configured to emit angled lighting beams S2.

Continuing with Fig. 1, in the surgical microscope, the coaxial lighting unit 21 and the angled lighting unit 22 can be enabled separately, or can be enabled simultaneously. The coaxial lighting unit 21 and the angled lighting unit 22 can be turned on or off by using different start switches or buttons.

In the embodiments of the present disclosure, in some surgical scenarios, the coaxial illumination (0° illumination) and the angled illumination (e.g. field illumination from 5° to 7°) can be enabled separately, and the two coaxial lighting paths corresponding to the left and right eyes are symmetrical with respect to the main optical axis of the surgical microscope system. In some other surgical scenarios, the coaxial illumination and the angled illumination can be enabled simultaneously. In an implementation, in a case of simultaneous illumination with the coaxial lighting unit 21 and the angled lighting unit 22, the light intensity ratio of the coaxial illumination to the angled illumination is set to 4: 15, and the angle between the angled lighting beam S2 refracted by the objective lens 11 and the coaxial lighting beam S1 refracted by the objective lens 11 is between 5° and 7°, which can play the role of balancing the optical spot energy, broadening the field of view, and increasing the light brightness.

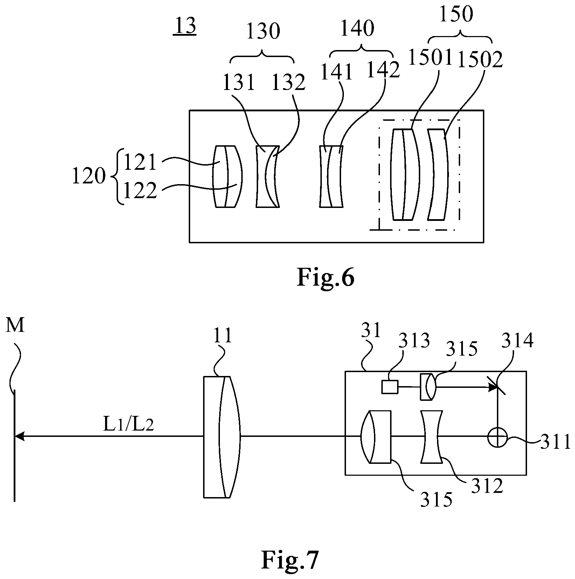

Fig. 6 is a schematic diagram of a zoom unit provided by embodiments of the present disclosure.

As shown therein, the zoom unit 13 includes a front fixed set 120, a zoom set 130, a compensation set 140, and a rear fixed set 150. The front fixed set 120, zoom set 130, and the compensation set 140 are all cemented lenses. By way of example, the front fixed set 120 includes a third lens 121 and a fourth lens 122 arranged sequentially along a main optical axis from the image side to the object side; the zoom set 130 includes a fifth lens 131 (with a negative focal power) and a sixth lens 132 (with a negative focal power) arranged sequentially along the main optical axis from the image side to the object side; the compensation set 140 includes a seventh lens 141 (with a negative focal power) and an eight lens 142 (with a negative focal power) arranged sequentially along the main optical axis from the image side to the object side. The rear fixed set 150 includes two lenses, namely a cemented lens 1501 and a meniscus lens 1502 arranged sequentially along the main optical axis from the imaging side to the object side.

According to the embodiments of the present disclosure, the front fixed set 120 has a positive focal power; the zoom unit 130 has a negative focal power; the compensation set 140 has a negative focal power; the rear fixed set 150 has a positive focal power. Therefore, the zoom unit 13 is of a positive-negative-positive structure. In the embodiments of the present disclosure, the zoom unit 13 has a zoom ratio up to 1:6, a change range from 0° to 7.4° in terms of field of view, and a change range from 3.4mm to 18mm in terms of entrance pupil diameter.

In the embodiments of the present disclosure, the compensation set 140 is configured to compensate for an off-axis aberration generated during zoom movement, which can effectively achieve an aberration balance between respective focal sections and ensure the clarity of the image in the case of different focal lengths. The cooperation of the zoom set 130 and the compensation set 140 enables continuous afocal zooming, and the system has the characteristics of a large zoom ratio, and a continuously changing field of view, which can cause the surgical microscope to implement continuous afocal zooming, thus eliminating the discomfort for the observer (e.g. a surgeon) caused by discontinuities in field of view.

In the embodiments of the present disclosure, as shown in Fig. 1, the objective lens 11 includes a first lens 111 and a second lens 112 cemented together; a surface of the lens close to the object surface is an object side surface, and a surface of the lens close to the image surface is an image side surface; the object side surface of the first lens 111 is a flat surface, and the image side surface of the first lens is a concave surface; the object side surface of the second lens 112 is a convex surface, and the image side surface of the second lens 112 is a convex surface; the first lens 111 has a refractive index of n1, and the second lens 112 has a refractive index of n2; the first lens 111 has an Abbe constant of v1, and the second lens has an Abbe constant of v2; wherein, n1> n2, and v1 < v2.

The refractive index is a ratio of a speed of light in a vacuum to a speed of light in a medium to describe an ability of a material to refract light. Different materials are varied in refractive index. A high refractive index of a material indicates a powerful ability thereof to refract incident light. An Abbe constant is an index for indicating a dispersion ability of a transparent medium. A great dispersion of a transparent medium indicates a small Abbe constant; and a minor dispersion of a transparent medium is accompanied with a large Abbe constant.

Considering that a white light source is used for imaging, dispersion is generated due to different refractive indices of different colored light. In the case, different colored light has different optical paths, and the final result is an aberration, also referred to as chromatic aberration, caused by different optical paths of different colored light. In the embodiments of the present disclose, the first lens 111 is a plano-concave lens, the second lens 112 is a concave-concave lens, the first lens 111 and the second lens 112 are cemented, and the refractive index n1 of the first lens 111 is greater than the refractive index n2 of the second lens 112. Such arrangement can increase the amount of incident light. Moreover, since a chromatic aberration is also introduced, the Abbe constant v1 of the first lens 111 is less than the Abbe constant v2, to reduce the dispersion effect and eliminate the chromatic aberration.

In the embodiments of the present disclosure, as shown in Fig. 1, the lighting module 02 further includes a stray light absorption unit 23. The stray light absorption unit 23 is disposed on a side of the dichroic beam splitting lens 12 facing away from the coaxial lighting unit 21 and in the traveling path of the lighting beams passing through the dichroic beam splitting lens 12 in the coaxial lighting beams S1.

In the embodiments of the present disclosure, the lighting module 02 is further provided with a stray light absorption unit 23 for preventing interference of stray light, which is configured to collect stray light beams passing through the dichroic beam splitting lens 12 among the lighting beams. In the case, the dichroic beam splitting lens 12 on the side facing away from the coaxial lighting unit 21 is provided with a stray light absorption unit 23, such that coaxial lighting beams S1 passing through the dichroic beam splitting lens 12 can be absorbed by the stray light absorption unit 23, to accomplish the purpose of eliminating the stray light. By way of example, the stray light absorption unit 23 may be of an ellipsoidal bowl structure which is formed of a light absorbing material at its inside, and coated with a light absorbing film at its inner surface. The curvature of the ellipsoidal bowl is determined by its distances to the dichroic beam splitting lens 12 and the objective lens 11. In the embodiments of the present disclosure, the stray light absorption unit 23 can be designed as an outer wall of a mechanical frame of the objective lens 11.

Fig. 7 is a schematic diagram of an optical path of a scanning unit provided by embodiments of the present disclosure.

In the embodiments of the present disclosure, returning to Fig. 1 and referring to Fig. 7, the surgical microscope system may further include an OCT imaging module 03 comprised of a scanning unit 31 and an OCT image acquisition unit 32. The OCT image acquisition unit 32 is disposed on a main optical axis between the zoom unit 13 and the dichroic beam splitting lens 12, and the scanning unit 31 is disposed between a main optical axis between the dichroic beam splitting lens 12 and the objective lens 11.

In the embodiments of the present disclosure, the OCT imaging module 03 is configured to collect and display OCT images of an observed eye. OCT is a routine ophthalmic examination method mainly for examining an anterior and a posterior segment (including the fundus) of the eye. In the embodiments of the present disclosure, the OCT imaging module 03 is disposed in the optical path of the surgical microscope system, and as the scanning unit 31 and the coaxial lighting unit 21 are disposed on the same side of the dichroic beam splitting lens 12, the optical axis L2 of the scanning beams S5 reflected by the dichroic beam splitting lens 12 after emitted from the scanning unit 31 coincides with the main optical axis L1. The scanning unit 31 further includes a scanning galvanometer 311, a focus tuning lens 312, an OCT system light source 313, a second reflector 314, a plurality of focusing lenses 315, a controller, and the like. The scanning galvanometer 311 is configured to scan the examined eye, and the focus tuning lens 312 is configured to scan the examined eye. The focus tuning lens 312 is a negative lens, which is configured to finely tune the axis. As a joint effect of the scanning galvanometer 311 and the focus tuning lens 312, OCT tomographic imaging of the observed object surface M can be implemented.

The OCT image acquisition unit 32 is disposed on the main optical axis between the dichroic beam splitting lens 12 and the zoom unit 13. The OCT image acquisition unit 32 is a high speed Charged-coupled Device (CCD) camera. In an embodiment, the dichroic beam splitting lens 12 on the side facing the OCT image acquisition unit 32 is coated with a reflective film, to split the beams passing through the dichroic beam splitting lens 12 among the beams from the observed object surface M into two paths (the two paths of the beams are different in traveling direction), where: one path of beams is reflected by the reflective film of the dichroic beam splitting lens 12 and then enters the OCT image acquisition unit 32, to form an OCT tomographic image; and the other path of beams directly enters the zoom unit 13 along the direction of the main optical axis L1, to form a microscope image. Alternatively, in a further embodiment, a beam splitting component similar to the beam splitter 14 can be additionally provided between the dichroic beam splitting lens 12 and the zoom unit 13, to split the beams passing through the dichroic beam splitting lens 12 among the beams from the observed object surface M into two paths (the two paths of the beams are different in traveling direction), where: one path of beams enters the OCT image acquisition unit 32, to form an OCT tomographic image; and the other path of beams directly enters the zoom unit 13 along the main optical axis L1, to form a microscope image. According to the embodiments of the present disclosure, since the OCT image acquisition unit 32 is disposed on the main optical axis between the dichroic beam splitting lens 12 and the zoom unit 13, the size of the image displayed on the CCD interface of the OCT image acquisition unit 32 can be varied with the magnification of the surgical microscope; and since the observed object surface M and the CCD image surface are conjugate surfaces, an OCT image displayed in real time on the CCD interface can be consistent with the microscope image of the observed object surface M observed through the eyepiece set 16. Therefore, the OCT image displayed by the OCT image acquisition unit 32 on the CCD interface is consistent with the observed object surface M observed by an observer (e.g. a surgeon) through the eyepiece set 16 in terms of imaging magnification. In this way, as the microscope image observed by the surgeon through the eyepiece set 16 during the operation is consistent with the OCT image observed by the assistant through the CCD interface, the purpose of intraoperative teaching can also be accomplished.

According to the embodiments of the present disclosure, the microscope image observed through the eyepiece set 16 of the surgical microscope is consistent with the surgical image acquired by the image acquisition unit 17 and the OCT image acquired by the OCT image acquisition unit 32 in terms of image content and field of view, and the microscope image observed through the eyepiece set 16 is consistent with the OCT image acquired by the OCT image acquisition unit 32 and displayed on the CCD interface in terms of magnification, in addition to image content and field of view. The surgical images acquired by the image acquisition unit 17 can be used for postoperative archiving and analysis.

On the basis of the same idea, embodiments of the present disclosure further provide a surgical microscope including a surgical microscope system provided by the embodiments described above. The surgical microscope has the effects of the surgical microscope system described in the above implementations. For the similarities omitted herein, reference could be made to the above explanation and description of the surgical microscope system.

## Claims

1. A surgical microscope system, comprising a microscope imaging module (01) and a lighting module (02);
the microscope imaging module (01) comprises an objective lens (11), a dichroic beam splitting lens (12), a zoom unit (13), a beam splitter (14), a lens tube (15) and an eyepiece set (16) disposed along a main optical axis (L1); beams from an observed object surface (M) pass sequentially through the objective lens (11), the dichroic beam splitting lens (12) and the zoom unit (13) and are then split by the beam splitter (14) into first beams (S3) and second beams (S4), wherein the first beams (S3) pass sequentially through the lens tube (15) and the eyepiece set (16) along the main optical axis (L1), which is configured to be observed by an observer; the microscope imaging module (01) further comprises an image acquisition unit (17) disposed on a traveling path of the second beams (S4) and configured to acquire surgical images; wherein the first beams (S3) and the second beams (S4) are different in traveling direction;
the lighting module (02) comprises a coaxial lighting unit (21) and an angled lighting unit (22) which are located on a side of the dichroic beam splitting lens (12) facing the objective lens (11); the coaxial lighting unit (21) comprises a first light source (211) and a first field stop (212) disposed between the first light source (211) and the dichroic beam splitting lens (12), and coaxial lighting beams emitted from the first light source (211) pass through the first field stop (212), are reflected by the dichroic beam splitting lens (12), then pass through the objective lens (11), and reach the observed object surface (M) along a direction of the main optical axis (L1) and form a first optical spot; angled lighting beams emitted from the angled lighting unit (22) are reflected by the dichroic beam splitting lens (12), then pass through the objective lens (11), and reach the observed object surface (M) along a direction including a preset angle with respect to the main optical axis (L1) and form a second optical spot; the first optical spot is adjustable in size.

2. The surgical microscope system of claim 1, wherein the first field stop (212) is provided thereon with a plurality of clear apertures (O) differing in size, and different clear apertures (O) on the first field stop (212) are selected to form first optical spots differing in size.

3. The surgical microscope system of claim 2, wherein the first field stop (212) comprises one of the following:
a first disc-shaped stop (2121), wherein the first disc-shaped stop (2121) is provided thereon with a plurality of clear apertures (O) differing in size and having centers located on a same circumference with a center of the first disc-shaped stop (2121) being the center;
a rectangular stop (2122), wherein the rectangular stop (2112) is provided thereon with a plurality of clear apertures differing in size and having centers located on a same straight line;
a plurality of second disc-shaped stops (2123) arranged in an overlaid manner, wherein each of the second disc-shaped stops (2123) is provided thereon with a clear aperture, the different second disc-shaped stops (2123) are provided with clear apertures differing in size, and centers of a plurality of clear apertures corresponding to the plurality of second disc-shaped stops (2123) in an overlaid state are located on a same axis.

4. The surgical microscope system of claim 1, wherein the coaxial lighting unit (21) and the angled lighting unit (22) can be enabled separately, or can be enabled simultaneously.

5. The surgical microscope system of claim 1, further comprising: an Optical Coherence Tomography (OCT) imaging module (03) comprising a scanning unit (31) disposed on a main optical axis (L1) between the zoom unit (13) and the dichroic beam splitting lens (12), and an OCT image acquisition unit (32) disposed on a main optical axis (L1) between the dichroic beam splitting lens (12) and the objective lens (11).

6. The surgical microscope system of claim 1, wherein the lens tube (15) is provided therein with a first lens set (151) located on an end of the lens tube (15) close to the eyepiece set (16), and a second lens set (152) located on an end of the lens tube (15) close to the zoom unit (13); the first lens set (151) comprises a meniscus lens, and the second lens set (152) comprises a cemented lens.

7. The surgical microscope system of claim 1, wherein the coaxial lighting unit (21) further comprises a fundus functional lens (213); the fundus functional lens (213) includes a light transmitting portion (P1) and a light shielding portion (P2), the light transmitting portion (P1) is disposed around the light shielding portion (P2), the fundus functional lens (213) is located between the first light source (211) and the first field stop (212), and the light shielding portion (P2) is located on an optical axis of the coaxial lighting beams emitted from the first light source (211); the light shielding portion (P2) has a light transmittance of T1<1%, and the light transmitting portion (P1) has a light transmittance of T2>99%.

8. The surgical microscope system of claim 1, wherein the lighting module (02) further comprises a stray light absorption unit (23); the stray light absorption unit (23) is disposed on a side of the dichroic beam splitting lens (12) facing away from the coaxial lighting unit (21) and on a traveling path of lighting beams passing through the dichroic beam splitting lens (12) in the coaxial lighting beams.

9. The surgical microscope system of claim 1, wherein the objective lens (11) comprises a first lens (111) and a second lens (112) cemented together; a surface of a lens close to an object surface is an object side surface, and a surface of the lens close to an image surface is an image side surface;
an object side surface of the first lens (111) is a flat surface, and an image side surface of the first lens (111) is a concave surface; an object side surface of the second lens (112) is a convex surface, and an image side surface of the second lens (112) is a convex surface; the first lens (111) has a refractive index of n1, and the second lens (112) has a refractive index of n2; the first lens (111) has an Abbe constant of v1, and the second lens (112) has an Abbe constant of v2; wherein n1>n2, and v1<v2.

10. A surgical microscope, comprising a surgical microscope system of any of claims 1-9.
